# EUROPÄISCHE PATENTSCHRIFT

(11) **EP 0 618 797 B1**
(45) Veröffentlichungstag und Bekanntmachung des Hinweises auf die Patenterteilung: **15.03.2000**
(21) Anmeldenummer: 93901748.9
(22) Anmeldetag: 22.12.1992
(51) Int. Cl.: A61K 31/365, A61K 35/78

(54) **BILOBALID ENTHALTENDE PHARMAZEUTISCHE ZUBEREITUNGEN ZUR BEHANDLUNG VON SPANNUNGS- UND ANGSTZUSTÄNDEN**
PHARMACEUTICAL PREPARATIONS CONTAINING BILOBALID FOR THE TREATMENT OF NERVOUS TENSION AND ANXIETY
PREPARATIONS PHARMACEUTIQUES RENFERMANT DU BILOBALIDE POUR LE TRAITEMENT DES ETATS DE TENSION NERVEUSE ET D'ANXIETE

(30) Priorität: 23.12.1991 DE 4142878
(43) Veröffentlichungstag der Anmeldung: 12.10.1994
(73) Patentinhaber: Dr. Willmar Schwabe GmbH & Co., 76227 Karlsruhe (DE)
(72) Erfinder: NÖLDNER, Michael, D-7514 Eggenstein (DE); CHATTERJEE, Shyam, S., D-7500 Karlsruhe 1 (DE)
(74) Vertreter: VOSSIUS & PARTNER
(86) Internationale Anmeldenummer: EP9202981
(87) Internationale Veröffentlichungsnummer: WO9312784

(56) Entgegenhaltungen:
- EP-A- 0 143 977
- GB-A- 2 023 421
- INVESTIGACION MEDICA INTERNACIONAL Bd. 17, Nr. 3, 1990, Seiten 130 - 141 DR. C. E. PIETRA SANTA V ET AL. 'EFICACIA DEL EXTRACTO ESTANDARIZADO DE GINKGO-BILOBA EGB 761* EN EL TRATAMIENTO DE LA INSUFICIENCIA VASCULAR CEREBRAL'
- LA PRESSE MEDICALE Bd. 15, Nr. 31, 25. September 1986, Seiten 1595 - 1604 D.M. WARBURTON 'PSYCHO-PHARMACOLOGIE CLINIQUE DE L'EXTRAIT DE GINKGO BILOBA'
- FORTSCHRITTE DER MEDIZIN Bd. 108, Nr. 29, 10. Oktober 1990, M]NCHEN Seiten 557 - 560 F. ECKMANN 'HIRNLEISTUNGSSTöRUNGEN BEHANDLUNG MIT GINKGO-BILOBA-EXTRAKT'
- CHEMICAL ABSTRACTS, vol. 113, no. 17, 22. Oktober 1990, Columbus, Ohio, US; abstract no. 151138d, M. TAKESHI ET AL. 'MANUFACTURE OF CHOCOLATE CONTAINING TERPENES AND FLAVONE GLYCOSIDES OF GINKGO LEAVE EXTRACT.' Seite 664 ;Spalte 2 ;

## Beschreibung

Über die Isolation eines Laktones mit der Formel C₁₅H₁₈O₈ aus den Blättern des Ginkgo biloba Baumes berichtete erstmals R.T. Major (Science 157, 1967, 1270-1273). K. Weinges und W. Bähr gaben der Substanz den Namen Bilobalid und untersuchten und beschrieben die physikochemischen Eigenschaften von Bilobalid und Derivaten (Liebigs Ann. Chem., 724, 1969, 214-216).
Nach einer gemeinsamen Publikation der Arbeitsgruppen von K. Nakanishi et.al., R.T. Major et. al. und K. Weinges et.al. (J. Amer. Chem. Soc. 93, 1971, 3544-3546) hat Bilobalid die Formel I

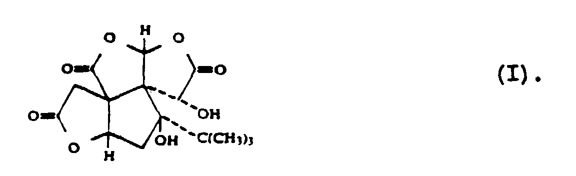

Seit 1965 werden Extrakte aus den Blättern des Ginkgo biloba Baumes therapeutisch zur Behandlung von zentralen und peripheren Durchblutungsstörungen eingesetzt. Diese Extrakte enthalten als Hauptbestandteile Flavonglycoside und sind u.a. auf diese Inhaltsstoffe standardisiert. Ein typischer Repräsentant dieser Gruppe ist 5,7,3',4'-tetrahydroxyflavono-3-O-alpha-rhamnopyranosyl-4-0-beta-D-(6'''-trans-coumaroyl)glycopyranosid der Formel II

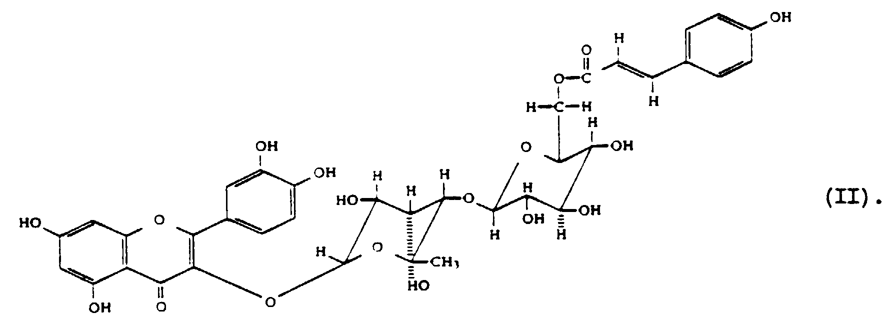

Diese Extrakte enthalten in der Regel auch geringere Mengen Bilobalid und Ginkgolide A, B, C und J.

Aus US-A-4,571,407 ist bekannt, daß Bilobalid zur Behandlung diverser degenerativer, neuronaler Erkankungen geeignet ist. Insbesondere hardelt es sich dabei um Neuropathien, Enzephalopathien und Myelopathien, die mit einem oder mehreren der folgenden Symptome verbunden sind: Paraesthesien, Paresen, abnorme Reflexe, Muskelatrophie, Muskelspasmen, Tremor, Kopfschmerzen, Sprach- und Hörstörungen, Schwindel, Bewußtseins- oder Koordinationsstörungen etc.

Es wurde nun überraschenderweise entdeckt, daß Bilobalid neben den bereits bekannten pharmakologischen Wirkungen auf degenerative neurologische Erkrankungen auch anxiolytische Aktivitäten besitzt. Damit sind erstmals psychopharmakologische Wirkungen von Bilobalid bekannt geworden.

Die Angst, die eigentlich einen nützlichen Schutzmechanismus des Menschen darstellt, da sie ihn häufig davor warnt sich bestimmten Gefahren auszusetzen, wird pathologisch, wenn sie ungezielt oder unverhältnismäßig stark ausgeprägt ist. Angstpatienten zeigen u.a. Symptome wie innere Erregung, Unruhe, Lustlosigkeit und haben häufig Einschlaf- und Durchschlafstörungen.

Pathologische Angst ist ein häufig auftretendes Symptom, dem oftmals keine ausreichende Beachtung geschenkt wird, obwohl Angst häufig die Ursache für sogenannte psychosomatische Erkrankungen ist. Die Behandlung pathologischer Angstzustände geschieht bisher entweder durch Psychotherapie und/oder medikamentöse Therapie mit anxiolytisch wirksamen Arzneimitteln.

Die pharmakologisch wirksamen Anxiolytika entstammen hauptsächlich der Gruppe der Benzotiazepine (z.B. Diazepam). Neuere Anxiolytika sind pharmakologisch als 5HT₁ₐ Agonisten (z.B. Buspiron) charakterisiert worden. Überraschenderweise zeigt Bilobalid in verschiedenen Tiermodellen Aktivitäten, die weitgehend der Wirkung von Diazepam oder Buspiron entsprechen.

Gegenstand der Erfindung ist somit die Verwendung von Bilobalid oder mit Bilobalid angereicherten Ginkgo biloba Extrakten zur Herstellung eines Arzneimittels zur Behandlung oder Prophylaxe von Angst- und Spannungszuständen sowie von depressiven Erkrankungen, die jeweils nicht durch zerebrovaskuläre Insuffizienz hervorgerufen worden sind, wenn mit Bilobalid angereicherte Ginkgo biloba Extrakte verwendet werden.

Als Tiermodelle zum Nachweis einer anxiolytischen Aktivität dienen 1. das "Elevated Plus Maze", 2. die "Hell-Dunkel-Box" sowie 3. der "angstinduzierte Temperaturanstieg".
1. Beim "Elevated Plus Maze" werden Ratten in das Zentrum des Labyrinths gesetzt und während 5 Minuten auf ihr Verhalten untersucht. Kontrolltiere zeigen normales Explorationsverhalten, halten sich aber überwiegend im "sicheren" Bereich der dunklen Seitenarme des Labyrinths auf. Anxiolytisch wirksame Pharmaka verlängern die Aufenthaltedauer der Versuchstiere und erhöhen die Häufigkeit des Wechselns in die "unsicheren" offenen Seitenarme des Labyrinths. (Lister, R.G. [1990] Ethologically-based animal models of anxiety disorders; Pharmac. Ther., 46, 321-340).
2. Bei der "Hell-Dunkel-Box" werden die Mäuse oder Ratten in das helle Feld gesetzt und während 3 Minuten auf ihr Verhalten beobachtet. Kontrolltiere zeigen neben normalem Explorationsverhalten eine Präferenz für die dunkle Box. Anxiolytika ärdern das Verhalten der Tiere dahingehend, daß die Aufenthaltdauer in hellen Feld verlängert wird (Lister, R.G. [1990] Ethologically-based animal models of anxiety disorders; Pharmac. Ther., 46, 321-340).
3. Beim Modell der "angstinduzierten Temperaturerhöhung" werden Mäuse in Gruppen von 18 Tieren/Käfig zusammengesetzt und der Reihe nach aus dem Käfig genommen, für ca. 20 Sekunden gehalten und wieder in den gleichen Käfig zurückgesetzt, wobei bei den ersten drei und bei den letzten drei Mäusen die Körpertemperatur rektal gemessen wird. Die Temperatur der letzten Mäuse ist im Durchschnitt 1-1,5° C höher als die der ersten drei Tiere. Dieser induzierte Temperaturanstieg ist durch am Menschen anxiolytisch wirksame Arzneimittel hemmbar. (Lecci, A. et.al. [1990] Pharmacological validation of a novel animal model of anticipatory anxiety in mice; Psychopharmacology, 101, 225-261).

Die Wirksamkeit von Bilobalid wird durch die folgerden Experimente demonstriert.

### Experiment 1a: Elevated Plus Maze

Männliche Sprague Dawley Ratten im Gewicht von 150-250 g erhielten Bilobalid peroral in Dosierungen von 5, 10 oder 20 mg/kg in 10 ml 0,2 %iger Agarsuspension einmalig 60 Minuten vor dem Test. Kontrolltiere erhielten nur Agar, Referenztiere Diazepam oder Ethanol peroral verabreicht. Sowohl nach Einzelgabe als auch nach wiederholter Bilobalidgabe (5 Tage 1 x täglich) war eine hochsignifikante Wirkung vorhanden.

### Experiment 1b: Elevated Plus Maze

In einer im Vergleich zum vorsteherden Modell modifizierten Versuranordnung wurden die Ratten zu Beginn des Experimentes nicht in das Zentrum des Labyrinthes, sondern an das äußerste Ende eines offenen Seitenarmes mit dem Gesicht nach außen gesetzt. Der Versuch wurde an zwei aufeinanderfolgenden Tagen mit den gleichen Tieren durchgeführt. Die Ratten zeigen am 2. Versuchstag eine wesentlich verkürzte Aufenthaltsdauer auf den offenen Armen und korrespondierend ein reduziertes Wechseln der Labyrintharme. Dies ist ein als Lernen zu interpretierendes Phänomen, da die Ratten am 2. Versuchstag das Labyrinth kennen urd schnell ihr Ziel erreichen.

Es konnte nun in dieser Versuchsanordnung gezeigt werden, daß die Standardsubstanz Diazepam die Aufenthaltsdauer im offenen Arm verlängert, was als anxiolytische Wirksamkeit interpretiert wird, daß die Ratten aber trotz Trainings die Seitenarme so häufig wechseln, wie untrainierte Kontrollratten.

Bilobalid-behandelte Ratten zeigen ebenfalls eine Verlängerung der Aufenthaltsdauer in offenen Arm, aber auch eine reduzierte Wechselfrequenz zwischen den Labyrintharmen. Dieses Ergebnis deutet darauf hin, daß Bilobalid im Gegensatz zu Diazepam das Lernverhalten trotz anxiolytischer Wirksamkeit nicht beeinflußt.

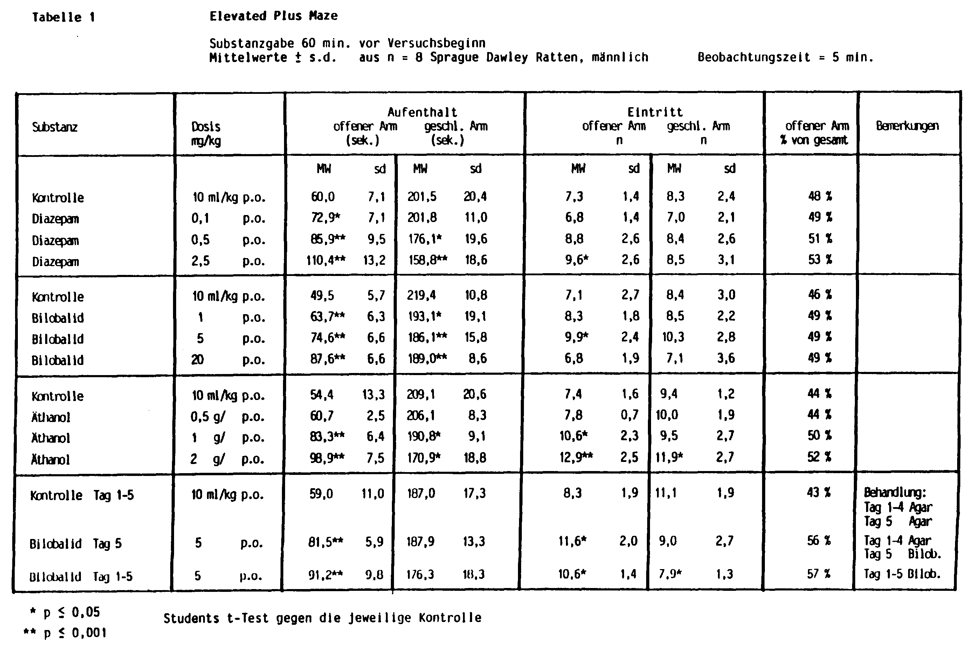

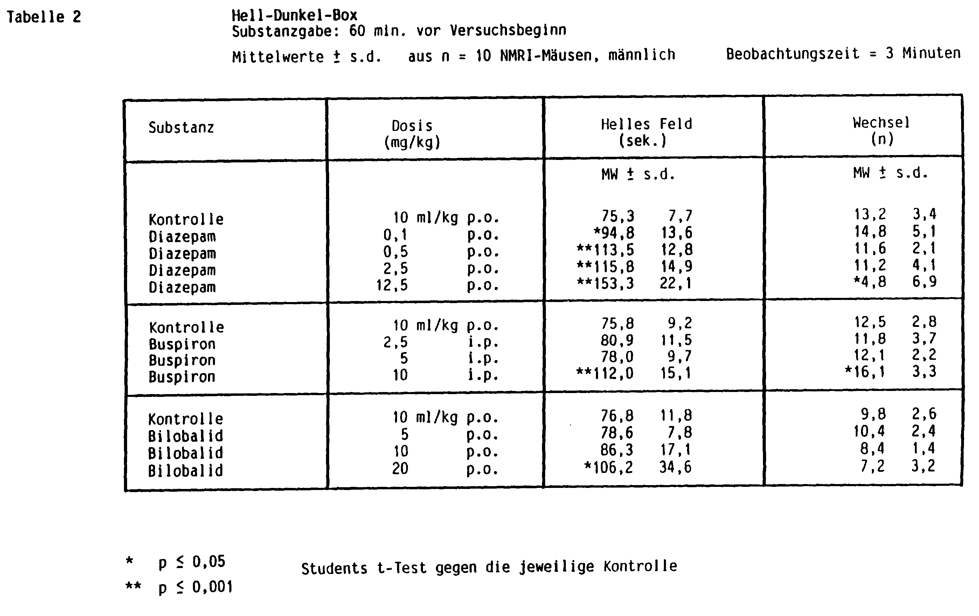

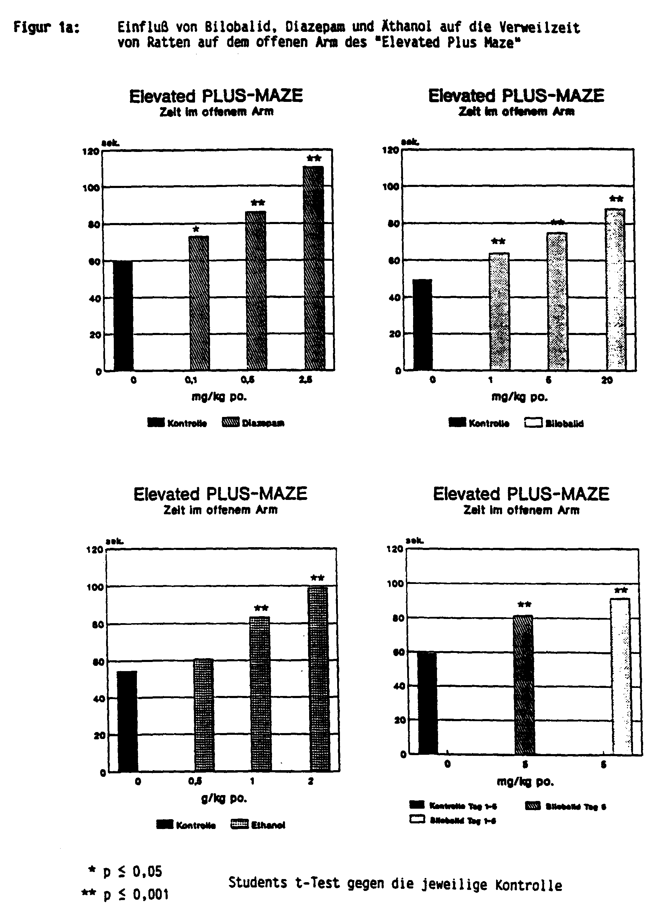

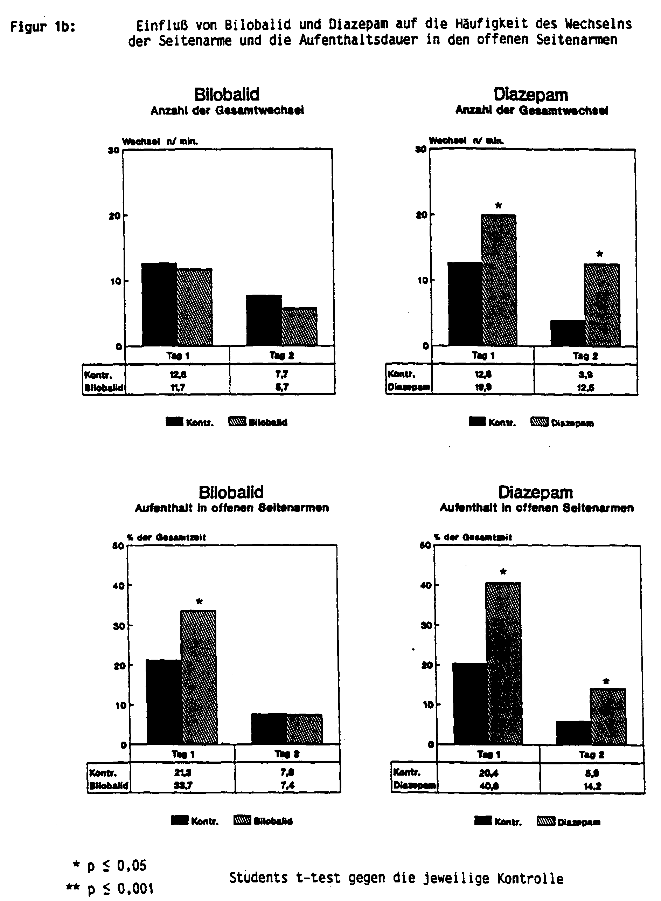

Die Ergebnisse dieser Versuche sind in Tabelle 1 und in den Figuren 1a und 1b dargestellt.

### Experiment 2: Hell-Dunkel-Box

In diesen Experiment erhielten männliche NMRI-Mäuse im Gewicht von 20-30 g, Bilobalid in Dosen von 1,5 und 20 mg/kg 1 x täglich, 60 Minuten vor Durchführung des Versuches peroral per Schlundsonde verabreicht. Kontrolltiere erhielten Agar-Suspension (0,2 %ig), Referenztiere Diazepam, Buspiron oder Ethanol. Bilobalid zeigt in diesen Modell ebenso wie die Referenzsubstanzen Diazepam, Buspiron und Ethanol eindeutige, statistisch signifikante Effekte im Sinne einer anxiolytischen Wirkung.

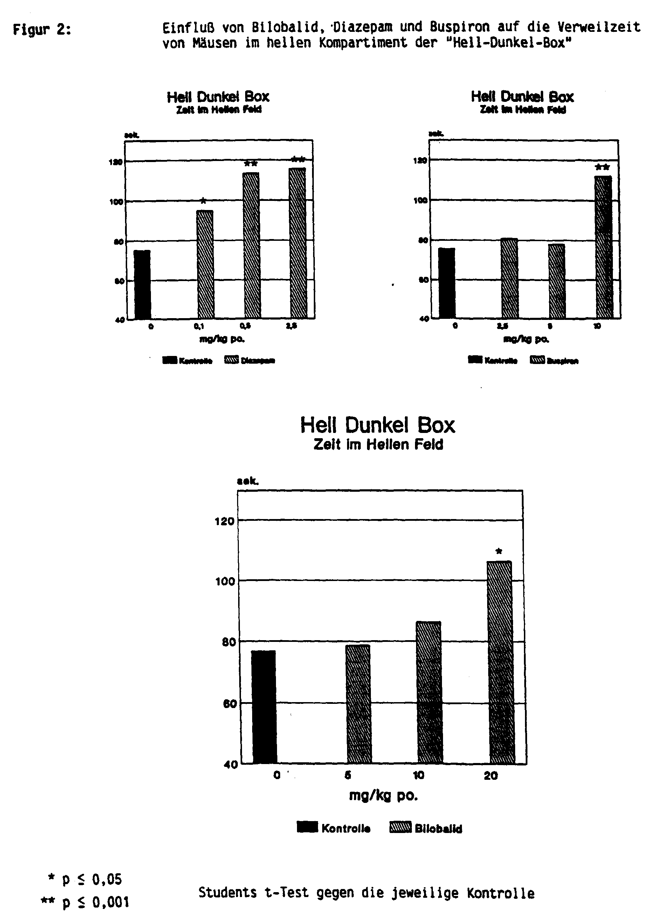

Die Ergebnisse dieser Studien sind der Tabelle 2 zu entnehmen und in Figur 2 dargestellt.

### Experiment 3: Temperatur-Versuch

In diesen Modell zeigt Bilobalid in einer Dosierung von 20 mg/kg peroral eine deutliche, statistisch signifikante Reduktion des streßbedingten Temperaturanstiegs. Die Wirkung von Bilobalid ist der der Referenzsubstanzen Diazepam urd Mephenesin vergleichbar.

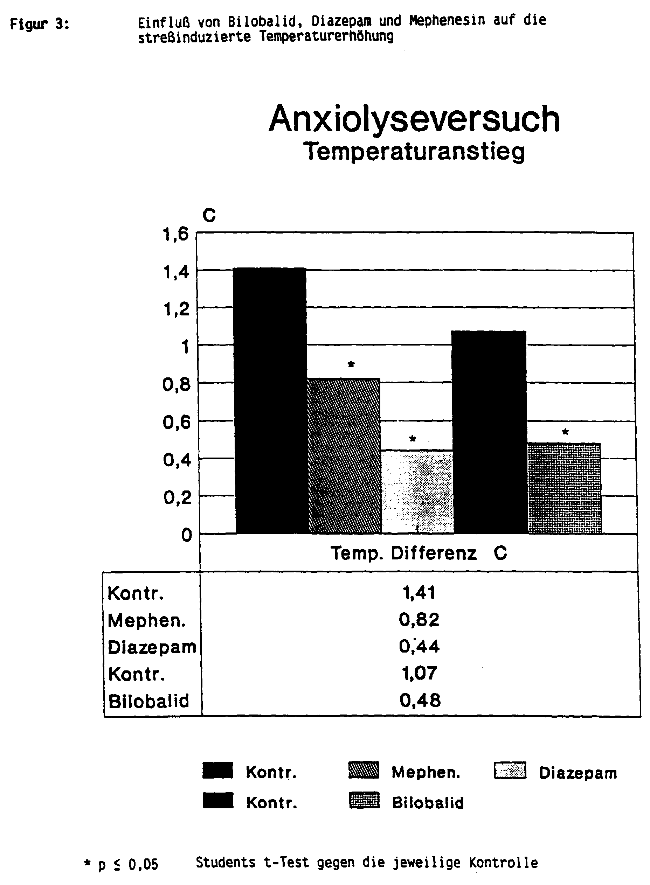

Die Ergebnisse sind in der Figur 3 dargestellt.

Bilobalid zeigt in allen hier gewählten Versuchsmodellen pharmakologische Wirkungen, die auf eine anxiolytische Aktivität hinweisen.

Bilobalid kann in Form von üblichen Arzneimitteln, z.B. Lösungen, Dragees, Tabletten, Kapseln, Injektions- oder Infusionslösungen, oral oder parenteral, z.B. intramuskulär oder intravenös gegeben werden. Die Dosis hängt ab von der Schwere der Erkrankung und dem Gewicht des Patienten. Dragees können morgens und abends nach den Mahlzeiten gegeben werden. Als Tagesdosen werden bei den normalen Arzneiformen 5 bis 40 mg Bilobalid und bei parenteraler Applikation 0,5 bis 5 mg Bilobalid gegeben.

Bilobalid kann z.B. nach der von K. Weinges und W. Bähr, Justus Liebigs Ann. (Chem., 724 (1969), 214-216, angegebenen Methode aus den Blättern von Ginkgo biloba isoliert werden.

Zur Herstellung von Bilobalid enthaltenden Arzneimitteln können die üblichen Träger- und Zusatzstoffe verwendet werden. Übliche Trägerstoffe sind beispielsweise Wasser, physiologische Kochsalzlösung, Alkohole, Polyäthylenglykole, Glycerinester, Gelatine, Kohlenhydrate wie Lactose und Stärke, Magnesiumstearat, Talcum. Übliche Zusatzstoffe sind beispielsweise Konservierungsmittel, Sterilisierungsmittel, Gleitmittel, Netzmittel und Emulgatoren, Farbstoffe, Geschmackskorrigentien und Aromastoffe. Die Answahl der Träger- und Zusatzstoffe hängt davon ab, ob die erfindungsgemäßen Zubereitungen enteral oder parenteral appliziert werden sollen.
1. Tabletten mit reinem Bilobalid
   Zur Herstellung von Tabletten mit jeweils 100 mg Einzelgewicht, die 5 mg Bilobalid enthalten, benötigt man
   - 5 g: Bilobalid
   - 58,5 g: Lactose
   - 18 g: mikrokristalline Cellulose
   - 18 g: Maisstärke
   - 0,5 g: Magnesiumstearat

   Die ersten vier Bestandteile werden gemischt, granuliert und nach Zugabe von Magnesiumstearat auf einer Tablettiermaschine zu Tabletten verpreßt.
2. Tabletten mit Bilobalid enthaltendem Ginkgo-Extrakt
   Bei Verwendung eines an Bilobalid angereicherten Extraktes aus Ginkgo ergibt sich folgende Rezeptur:
   - n g: Ginkgo-Extrakt, entsprechend 5 mg Bilobalid
   - (200-n) g: Lactose
   - 25 g: mikrokristalline Cellulose
   - 24 g: Maisstärke
   - 1 g: Magnesiumstearat

   Die ersten vier Bestandteile werden gemischt, granuliert und nach Zugabe von Magnesiumstearat auf einer Tablettiermaschine zu Tabletten von 250 mg Einzelgewicht verpreßt.
3. Kapseln
   - 7 g: Bilobalid
   - 75 g: Lactose
   - 20 g: Maisstärke

   Die Bestandteile werden homogen gemischt und in üblicher Weise zu Kapseln mit einem Füllgewicht von 100 mg verarbeitet.
4. Injektionsampullen
   Zur Herstellung von Injektionsampullen mit jeweils 2 ml Inhalt, die 0,5 mg Bilobalid enthalten, benötigt man
   - 0,25 g: Bilobalid
   - 9 g: Natriumchlorid
   - ad 100 g: Aqua bidest

   Die ersten beiden Bestandteile werden unter leichtem Erwärmen urd Rühren in Wasser gelöst. Die Lösung wird steril filtriert und in 2 ml Ampullen abgefüllt.
5. Flüssige orale Arzneiform
   - 5 g: Bilobalid
   - 10 g: Aromassenz
   - 5 g: Natriumsaccharinat
   - 400 g: Äthylalkohol
   - 580 g: aqua dest. oder vollentsalztes Wasser

   Die ersten drei Bestandteile werden im Gemisch aus Äthanol und Wasser gelöst. Die erhaltene Lösung wird in 100 ml Fläschchen abgefüllt. Die Einzeldosis beträgt 1 ml.

## Patentansprüche

1. Verwendung von Bilobalid oder mit Bilobalid angereicherten Gingko biloba Extrakten zur Herstellung eines Arzneimittels zur Behandlung oder Prophylaxe von Angst- und Spannungszuständen und depressiver Erkrankungen, wobei die genannten Zustände bzw. Erkrankungen nicht durch zerebrovasculäre Insuffizienz hervorgerufen werden, wenn mit Bilobalid angereichste Gingko biloba Extrakte verwendet werden.

## Claims

1. Use of bilobalid or of Gingko biloba extracts enriched with bilobalid for the preparation of a pharmaceutical composition for the treatment or prophylaxis of anxiety, nervous tension and depressive diseases, said conditions or diseases not being induced by cerebrovascular insufficiency if Gingko biloba extracts enriched with bilobalid are used.

## Revendications

1. Utilisation de bilobalide ou d'extraits de Gingko biloba enrichis en bilobalide pour préparer un médicament pour le traitement ou la prophylaxie d'états d'anxiété et de tension nerveuse et de maladies dépressives, les états ou maladies cités n'étant pas provoqués par une insuffisance cérébrovasculaire, lorsque des extraits de Ginkgo biloba enrichis en bilobalide sont utilisés.
